# EUROPEAN PATENT APPLICATION

(11) **EP 2 774 971 A1**
(43) Date of publication of application: **10.09.2014**
(21) Application number: 14158309.6
(22) Date of filing: 07.03.2014
(51) Int. Cl.: C10G 1/00, C10G 19/073, C10G 27/04, C10G 27/14, C07C 27/10, C07C 67/39, C10L 1/02

(54) **Process for the upgrading of gaseous products obtained from thermal processing of biomass**

(30) Priority: 08.03.2013 FI 20135225
(71) Applicant: UPM-Kymmene Corporation, 00100 Helsinki (FI)
(72) Inventor: Asikkala, Janne, 53950 Lappeenranta (FI); Gutierrez, Andrea, 53200 Lappeenranta (FI); Kotilainen, Risto, 45100 Kouvola (FI)
(74) Representative: Boco IP Oy Ab

(57) **Abstract**

The present invention relates to a process for converting gaseous products, said process comprising the steps, where a feedstock comprising gaseous products obtained from thermal processing of biomass is subjected to oxidation in the presence of an oxidant, under conditions suitable for enacting said oxidation to yield an oxidation product, and subjecting the oxidation product to condensation in the presence of a basic catalyst to obtain bio-oil. The invention also relates to the use of bio-oil, obtainable by said process, as heating oil, as starting material in processes for producing fuels, fuel components, fine chemicals, chemical building-blocks, and solvents.

## Description

### FIELD OF THE INVENTION

The present invention relates to converting of gaseous products, particularly feedstock comprising gaseous products obtained from thermal processing of biomass, whereby the composition of said feedstock is altered, acidity is decreased and stability of is improved. The invention also relates to subjecting gaseous products obtained from thermal processing of biomass to oxidation under conditions suitable for oxidation to yield an oxidation product and subjecting said product to condensation under conditions suitable for condensation to provide converted bio-oil. The invention also relates to converted bio-oils obtainable by said process.

### BACKGROUND OF THE INVENTION

Bio-oils of varying properties and compositions are obtained using numerous methods and processes. Bio-oils may be obtained for example from biomass using any suitable thermal processing, such as pyrolysis and the like.

Pyrolysis is generally understood as the chemical decomposition of organic materials by heating in the absence or with limited supply of oxidizing agent such as air or oxygen. Pyrolysis can be used for converting biomass to pyrolysis oil which is an example of bio-oil. Commercial pyrolysis applications are typically either focused on the production of charcoal (slow pyrolysis) or production of liquid products (fast pyrolysis), the pyrolysis oil. Both the slow pyrolysis and the fast pyrolysis processes may be used for the manufacture of pyrolysis oil.

In fast pyrolysis solid biomass is thermally treated at the temperature typically ranging from 300 to 900 °C, and the residence time of the biomass in the pyrolyzer can be from a fraction of a second to a second. In pyrolysis of of lignocellulosic material most of the cellulose and hemicellulose and part of lignin typically disintegrate to form smaller and lighter molecules which are vapors at the pyrolysis temperatures. During cooling some of the vapors condense to form a liquid product, called pyrolysis oil.

Bio-oils are complex mixtures of chemical compounds, including reactive aldehydes and ketones. Said reactive compounds react with each other whereby complex molecules having higher molecular weight are formed and the viscosity of bio-oil is increased. For example biomass derived pyrolysis oil typically comprises water, light volatiles and non-volatiles. Further, pyrolysis oil has high acidity which typically leads to corrosion problems, substantial water content, and high oxygen content.

Wood-based pyrolysis oil is the product of pyrolysis of wood or forest residues and it contains typically carboxylic acids, aldehydes, ketones, carbohydrates, thermally degraded lignin, water, and alkali metals. The oxygen-containing compounds (typically 40-50 wt-%) and water (typically 15-30 wt-%) make pyrolysis oils chemically and physically unstable. Although pyrolysis oils have higher energy density than wood, they are acidic (pH∼2) and incompatible with conventional fuels. Furthermore pyrolysis oils have high viscosity and high solid content. Poor stability and high acidity are one of the key problems in utilizing the pyrolysis oil or storing for longer periods.

Due to its instability bio-oil is rapidly transformed to semisolid and gradually solid material, which is difficult to store or use for any further purposes. Thus, according to present practice it is necessary to process the bio-oils rapidly further in order to avoid the problems relating to stability.

Refining of bio-oils and particularly pyrolysis oils to provide fuel or fuel components is often very challenging due to the complex mixture of components of said bio-oil. For example pyrolysis oil typically consists of more than 200 identified compounds, which require very different conditions for converting them further to fuel components or precursors to fuel. Often this is carried out by hydroprocessing said pyrolysis oil over a hydrogenation catalyst in the presence of hydrogen. Since pyrolysis oil typically contains up to 50 wt% of oxygen, complete removal oxygen requires a substantial amount of hydrogen, even up to 1000 L/kg pyrolysis oil. The obtained light components are turned into gaseous products (hydrogen, methane, ethane, etc.) and heavy components are turned into coke and heavy oil. The heavy oil mixture needs further refinement to produce fuel fractions and this procedure requires high amounts of hydrogen and typically various different catalysts for obtaining the desired products.

Different alternatives have been studied for improving the quality of pyrolysis oil, such as catalytic fast pyrolysis, catalytic upgrading of the pyrolysis vapors, etc.

Despite the ongoing research and development relating to bio-oils, there is still a need to provide improved processes and methods for converting bio-oils to more valuable components in an efficient and economical way.

### SUMMARY OF THE INVENTION

The present invention relates to controlled oxidation of gaseous products obtained from thermal processing of biomass, in combination with condensation of the obtained oxidation products. The invention particularly relates to a process for converting gaseous product obtained from thermal processing of biomass, whereby composition of said product is altered, acidity is decreased and stability is improved. Particularly the present invention relates to a process for converting gaseous products, where a feedstock comprising gaseous products obtained from thermal processing of biomass is subjected to oxidation under conditions suitable for oxidation to yield oxidation product, and subjecting the oxidation product to condensation under conditions suitable for condensation to obtain bio-oil. With the process bio-oil, having improved stability and less complicated composition may be obtained, whereby the bio-oil is maintained in liquid form for long periods of time.

The present invention also provides bio-oil, which may be used as such as heating oil and as starting material in processes for producing fuels, fuel components, fine chemicals and chemical building-blocks for chemical production and solvents.

The process for converting gaseous products comprises the steps where, feedstock comprising gaseous products obtained from thermal processing of biomass is subjected to oxidation in gas phase in the presence of an oxidant selected from O₂, O₃, and H₂O₂ under conditions suitable for enacting said oxidation to yield oxidation product, and subjecting the oxidation product to condensation in the presence of a basic catalyst to obtain bio-oil.

Thus an object of the invention is to provide a process for effectively and economically retrieving bio-oil, whereby the viscosity of said bio-oil is decreased and stability improved.

Another object of the invention is to provide bio-oils, suitable for use as such or in the manufacture of more valuable components, particularly fuels and fuel components.

Still another object of the invention is to provide bio-oils based at least partly or totally on renewable starting materials for use as such or in the manufacture of more valuable components.

### DEFINITIONS

The term "hydroprocessing" refers here to catalytic processing of organic material by all means of molecular hydrogen.

The term "carbonyl compounds" refers here to all organic molecules containing one or more carbonyl groups, particularly aldehydes and ketones.

The term "chemical building-blocks" or "building-block chemicals" refer to chemical compounds useful as starting materials and intermediates for the manufacture of chemical and pharmaceutical final products. Examples of such chemical building-blocks are fumaric acid, furfural, glycerol, citric acid, treonin, propanic acid etc.

Transportation fuels refer to fractions or cuts or blends of hydrocarbons having distillation curves standardized for fuels, such as for diesel fuel (middle distillate from 160 to 380°C, EN 590), gasoline (150 - 210°C, EN 228), aviation fuel (160 to 300°C, ASTM D-1655 jet fuel), kerosene, naphtha, etc.

### BRIEF DESCRIPTION OF THE DRAWINGS

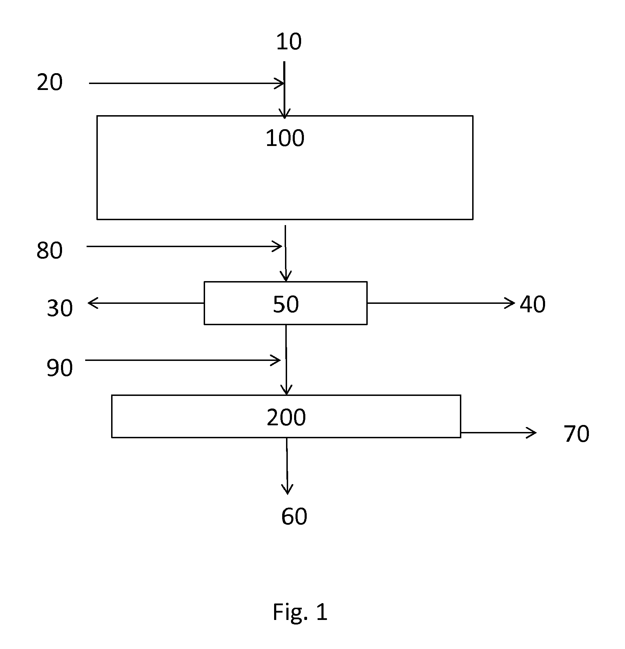

Fig. 1 is a schematic flow diagram representing one embodiment of the process for converting feedstock comprising gaseous products.

### DETAILED DESCRIPTION OF THE INVENTION

It was surprisingly found out that gaseous products obtained from thermal processing of biomass can be converted in an efficient manner to a homogeneous product mixture useful as valuable products, with a process where oxidation of the gaseous products and condensation of the oxidation product are carried out. In said process feedstock comprising gaseous products obtained from thermal processing of biomass, is subjected to oxidation under conditions suitable for oxidation to yield an oxidation product, and subjecting the oxidation product to condensation under conditions suitable for condensation to obtain bio-oil. The quality of bio-oils can be improved by treating bio-oil vapors with an oxidizing agent (oxygen, hydrogen peroxide, ozone), and subjecting the oxidation product to condensation.

In the oxidation step organic molecules may be degraded, whereby the oxidant (oxidation agent) forms carboxylic acid functions in the organic molecules, and further the oxidation breaks C-C bonds and can depolymerize complex molecules. The oxidation products are carboxylic acids, which are then subjected to condensation reaction in the second step to yiled longer chain oxygen containing hydrocarbons, particularly alcohols and/or saturated carbon chain (see scheme 1). For example Aldol condensation may be utilized is step 2.

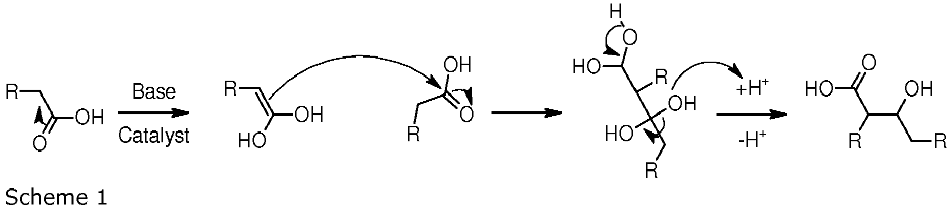

The oxidation step will produce more homogenous product from the gaseous products obtained from thermal processing of biomass, and the condensation step (referring here to condensation reaction step) increases the chain length of the oxidized compounds.

The obtained bio-oil may be used as starting material or feedstock in further refinement steps, as described for example in scheme 2 (hydrogenation), where the hydrogen consumption in the hydrogenation may be decreased significantly and more valuable long chain hydrocarbons may be obtained, particularly suitable as fuels or fuel components, such as transportation fuels.

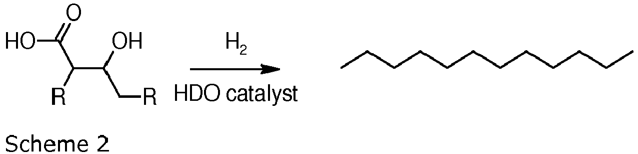

The process for converting feedstock comprising gaseous products comprises the steps, where a feedstock comprising gaseous products obtained from thermal processing of biomass, is subjected to oxidation in the presence of an oxidant selected from O₂, O₃ and H₂O₂, under conditions suitable for enacting said oxidation to yield an oxidation product, and subjecting the oxidation product to condensation in the presence of a basic catalyst to obtain bio-oil. The oxidation is suitably carried out in gas phase.

Figure 1 is a schematic diagram of a process in accordance with one embodiment of the invention. In this embodiment, in the first step feedstock 10, comprising gaseous products obtained from thermal processing of biomass, and oxidant 20 are fed to a reactor 100 wherein controlled oxidation is carried out. In a suitable embodiment the oxidant may be charged directly to a pipeline containing the gaseous feedstock whereby no separate oxidation reactor is needed (not shown in the figure). The reaction mixture 80 is subjected to separation in separation unit 50, where water 30 and char and gaseous components (such as CO₂, CO) 40 are separated and the oxidation product (gas or liquid depending on cooling) 90 is directed to reactor 200, where condensation reaction is carried out in the presence of a basic catalyst. Water 70 is separated either in connection with the condensation step or in a subsequent water removal step (not shown in the figure) and liquid bio-oil product 60 is obtained.

The gaseous products of the feedstock are any vapors or gaseous components or gaseous products obtained from any known thermal processing of biomass yielding vapors, and any combinations thereof. Said gaseous products are suitably obtained directly from said processing or treatment of biomass without cooling or condensing of the vapors whereby the temperature of the feedstock (such as pyrolysis vapors) is suitably 250-800°C. Said gaseous products may comprise pyrolysis vapors that may be obtained from any pyrolysis process of biomass, including slow pyrolysis, fast pyrolysis, catalytic pyrolysis, catalytic fast pyrolysis and hydropyrolysis (catalytic fast pyrolysis in the presence of hydrogen), suitably from fast pyrolysis.

Biomass may typically comprise virgin and waste materials of plant, animal and/or fish origin or microbiological origin, such as virgin wood, wood residues, forest residues, waste, municipal waste, industrial waste or by-products, agricultural waste or by-products (including also dung or manure), residues or by-products of the wood-processing industry, waste or by-products of the food industry, solid or semi-solid organic residues of anaerobic or aerobic digestion, such as residues from bio-gas production from lignocellulosic and/or municipal waste material, residues from bio-ethanol production process, and any combinations thereof. Biomass may include the groups of the following four categories: wood and wood residues, including sawmill and paper mill discards, municipal paper waste, agricultural residues, including corn stover (stalks and straw) and sugarcane bagasse, and dedicated energy crops, which are mostly composed of tall, woody grasses.

Suitably biomass is selected from non-edible sources such as non-edible wastes and non-edible plant materials. Particularly suitably said biomass comprises waste and by-products of the wood-processing industry such as slash, urban wood waste, lumber waste, wood chips, wood waste, sawdust, straw, firewood, wood materials, paper, by-products of the papermaking or timber processes, where the biomass (plant biomass) is composed of cellulose and hemicellulose, and lignin.

The gaseous products from pyrolysis refer particularly to complex mixtures of oxygen containing compounds (oxygenates), comprising typically water, light volatiles and non-volatiles. Said mixture is acidic, with a pH of 1.5- 3.8, and wood based mixture typically has pH between 2 and 3. The exact composition of the mixture depends on the biomass source and processing conditions. Typically gaseous pyrolysis product comprises CO₂, CO, H₂ and 20-30 % of water, 22-36 % of solids and pyrolitic lignin (including low molecular mass lignin and high molecular mass lignin), 8-12 % of hydroxyacetaldehyde, 3-8 % of levoglucosan, 4-8 % of acetic acid, 3-6 % of acetol, 1-2 % of cellubiosan, 1-2 % of glyoxal, 3-4 % of formaldehyde, and 3-6 % of formic acid by weight. Pyrolysis product typically also comprises other ketones, aldehydes, alcohols, furans, pyranes, sugars, organic acids, lignin fragments, phenolics, extractives and small amounts of inorganics.

The oxidant (oxidizing agent) is selected from O₂, O₃ and H₂O₂. The high temperature used in the process is enough to activate the oxidation and no additional catalyst is needed. In some embodiments inhibitors could be used to have better control of the oxidation. Oxidation reactions are exothermic which may require additional cooling of the vapors. This can be achieved for example by using water diluted H₂O₂.

Suitably the amount of oxidant, suitably O₂ or H₂O₂, is selected so that oxidation of the vapors to CO₂ does not take place. The partial oxidation will give more homogenous product mixture, mainly carboxylic acids, which can be processed further more easily than the conventional bio-oils. Even though the oxygen content in the molecules might be higher than that in conventional pyrolysis oil the oxygen functionality is easier to convert to fuels.

The amount of the oxidant is suitably 0.01-10 kg/kg of the feed, particularly 0.05-4 kg/kg of the feed.

The oxidation reaction is carried out at a temperature from 300 to 800°C, suitably from to 300-500°C.

The oxidation reaction is carried out under a pressure from 0.5 to 50 bar, suitably from 0.5 to 25 bar, particularly suitably from 0.5 bar to 10 bar, particularly when O₂, O₃ is used.

The residence time in oxidation reaction is 0.5 s to 300 s, particularly suitably from 1 to 100 s.

Due to the high oxidation temperature only small amounts of the oxidant are needed.

After the oxidation reaction, water is suitably separated using suitable means, such as evaporation, separation using a polar organic solvent, such as ethyl acetate, chlorinated solvents, methyl-tert-butylether etc.

The obtained oxidation product (referring here also to the reaction mixture obtained from the oxidation reaction) may directly be transferred as gas, or after cooling as liquid, without any purification or separation steps to the condensation step, or optionally one or more separation and purification steps may be carried out prior to the condensation reaction step. Any suitable mean for separation may be used, such as cyclonic separation, distillation, scrubbers including amine scrubbers and the like.

The condensation reaction step is carried out in the presence of a basic catalyst. Said catalyst is selected from silicates, aluminates, zeolites, alkali metal hydroxides, alkaline earth oxides, alkali metal oxides and rare earth oxides, suitably ThO₂, ZrO₂, ZnO₂, TiO₂, alkali ion-exchanged zeolites, alkali ion-added zeolites, alkali metal ions on alumina, alkali metal ions on silica, alkali metals on alkaline earth oxides, alkali metals and alkali metal hydroxides on alumina hydrotalcite, on chrysotile, on sepiolite, KF supported on alumina, lanthanide imide and nitride on zeolite may be used.

The following catalysts may also be used in the condensation step. Aldol additions and condensations are catalyzed by Ba(OH)2. Alkaline earth oxides, La2O3, and Zr02 are also active for the reaction in the following order: BaO > SrO>CaO > MgO>, La2O3 > Zr02. Zeolites are also active in aldol additions and condensations.

The condensation step is carried out at in the temperature range 300-450°C, suitably 350-400°C.

The condensation step is carried out under a pressure from NTP to 20 bar, suitably from 5 to 15 bar.

After the reaction, the different fractions of the product may be separated by fractionation based on boiling point (distillation) into light and heavy fraction. The fractions may not have the desired quality (for example of gasoline and diesel) and further processing will be required. These further processing could be e.g. hydroprocessing steps, such as hydrogenation, hydrodeoxygenation on conventional hydrotreating catalysts (NiMo/Al2O3, CoMo/Al2O3, NiW/Al2O3, etc.).

The process may be carried out a batch process, semi-batch process or a continuous process. In the process and in the oxidation and condensation steps any suitable reactors, equipment and configurations may be used, suitable for handling materials which may be corrosive. For example conventional reactors, tubular reactors, plug flow reactor as well as packed reactors, slurry reactors and fluid-bed reactors may be used. Suitably the process is a continuous process.

An oily, liquid bio-oil product is obtained having less acidity, lower amount of acids, lower amount of oxygen containing compounds, decreased viscosity, and it is a less complicated mixture of compounds. It has clearly increased stability and it is less corrosive.

With the process gaseous products obtained from thermal processing of biomass, particularly pyrolysis vapors can be upgraded in an effective and economic way.

The bio-oil product may be used as such for heating purposes as heating oil, where it provides clear advantages, such as higher heating value and higher quality than that of conventional bio-oils, such as pyrolysis oils. Due the improved stability and quality it may also be used as starting material in wider range of processes including processes for producing fuels, fuel components, particularly transportation fuels, fine chemicals and chemical building-blocks for chemical production, and solvents.

If desired bio-oil product may be subjected to any known hydroprocessing steps, and any pretreatment and purification steps if desired. Particularly in hydroprocessing simple hydrogenation conditions are sufficient and no complicated measures are needed, the consumption of H₂ is lower due to lower O₂ content in the bio-oil product, the yield are increased and better control of products is achieved.

In the process conventional cooling and gas/vapor condensing steps for obtaining liquid bio-oil, such as pyrolysis oil after thermal treatment or processing of biomass, can be avoided, very small amounts of the oxidant is needed, no evaporation steps for removing water from the bio-oil are needed, no heating of the feedstock is needed prior to the feeding to the oxidation step, the oxidation product may be transferred directly to the condensation step without any cooling or separation steps, whereby substantial amounts of energy can be saved, and further, simple equipment may be used for the process whereby the investments are low.

The oxidized product is more stable and can be easily converted in more valuable products. The catalysts used in the condensation are cheaper than metal catalyst and they don't need pretreatment.

## Claims

1. A process for converting gaseous products, wherein said process comprises the steps where a feedstock comprising gaseous products obtained from thermal processing of biomass is subjected to oxidation in the presence of an oxidant to yield an oxidation product, and subjecting the oxidation product to condensation carried out in the presence of a basic catalyst to obtain a bio-oil product.

2. The process according to claim 1, wherein the thermal processing is pyrolysis.

3. The process according to claim 1 or 2, wherein the feedstock is pyrolysis vapor or a combination of pyrolysis vapors.

4. The process according to any one of claims 1 - 3, wherein the temperature of the feedstock is 250-800°C.

5. The process according to any one of claim 1- 4, wherein the oxidant is selected from H₂O₂, O₂ and O₃.

6. The process according to any one of claims 1 - 5, wherein the oxidation is carried out at a temperature from 300 to 800°C, preferably from 300-500°C.

7. The process according to any one of claims 1 - 6, wherein the oxidation is carried out under a pressure from 0.5 to 50 bar.

8. The process according to any one of claims 1 - 7, wherein the basic catalyst is selected from silicates, aluminates, zeolites, alkalimetal hydroxides, alkaline earth oxides, alkali metal oxides, rare earth oxides, preferably Th02, Zr02, Zn02, Ti02, alkali ion-exchanged zeolites, alkali ion-added zeolites, alkali metal ions on alumina, alkali metal ions on silica, alkali metals on alkaline earth oxides, alkali metals and alkali metals hydroxides on alumina, on hydrotalcite, on chrysotile, on sepiolite, KF supported on alumina, lanthanide imide and nitride on zeolite.

9. The process according to any one of claims 1 - 8 wherein the condensation is carried out at a temperature from 300 to 450°C.

10. The process according to any one of claims 1 - 9, wherein the condensation stage is carried out under a pressure from NTP to 20 bar.

11. Use of the bio-oil product obtainable from the process according to any one of claims 1 - 10 as heating oil, as starting material in processes for producing fuels, fuel components, fine chemicals, chemical building-blocks, and solvents.
